(19) Europäisches Patentamt
European Patent Office
Office européen des brevets

(11) **EP 4 564 365 A1**

(12) **EUROPEAN PATENT APPLICATION**

(43) Date of publication:
**04.06.2025 Bulletin 2025/23**

(21) Application number: **24216166.9**

(22) Date of filing: **28.11.2024**

(51) International Patent Classification (IPC):
**G16H 30/40** (2018.01)    **G16H 20/40** (2018.01)
**G06N 3/045** (2023.01)    **G06T 7/00** (2017.01)

(52) Cooperative Patent Classification (CPC):
**G16H 30/40; G06N 3/045; G06T 7/0012;
G16H 20/40;** G06T 2207/10081; G06T 2207/10116;
G06T 2207/20081; G06T 2207/20084;
G06T 2207/30096

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.11.2023 US 202363604882 P
04.10.2024 US 202418907379**

(71) Applicant: **Siemens Healthineers International AG
6312 Steinhausen (CH)**

(72) Inventors:
• **PAYSAN, Pascal
4057 Basel (CH)**
• **SCHEIB, Stefan
8820 Wadenswil (CH)**
• **KUNZ, Dominik
4153 Reinach (CH)**
• **JUCHLER, Norman
8004 Zurich (CH)**

(74) Representative: **Mathisen & Macara LLP
Charta House
30-38 Church Street
Staines-upon-Thames TW18 4EP (GB)**

(54) **SYSTEMS AND METHODS FOR MEDICAL IMAGE EVALUATION AND VERIFICATION**

(57)    A method (200) for improving patient safety during medical treatment involves comparing medical images (502, 504) to verify patient identity. The method retrieves (210) a first medical image (502) of a patient and captures (220) a second image (504) using a medical imaging sensor. An artificial intelligence model (112) transforms these images (502, 504) into feature vectors in a latent space, where several features are identified and compared (230). The model predicts a distance between corresponding features in the two images (502, 504), associated with the likelihood that both images (502, 504) belong to the same patient. If this distance exceeds a set threshold (240), indicating a possible mismatch, a warning signal is sent to a radiotherapy computing device. This method helps prevent incorrect patient identification during medical procedures.

FIG. 5A

Processed by Luminess, 75001 PARIS (FR)

EP 4 564 365 A1

**Description**

## CROSS-REFERENCE TO RELATED APPLICATIONS

This application claims priority to U.S. Provisional Patent Application No. 63/604,882, filed on November 30, 2023, which is incorporated herein by reference in its entirety for all purposes.

## TECHNICAL FIELD

This application pertains generally to the field of medical imaging and patient safety in radiotherapy and, more specifically, to systems and methods for automatic verification of patient identity using artificial intelligence.

## BACKGROUND

Radiotherapy (RT) is a critical treatment modality for cancer, involving the precise delivery of ionizing radiation to target malignant tissues while sparing healthy tissues as much as possible. The process begins with careful planning based on high-resolution imaging, typically using Computed Tomography (CT), and is followed by daily setup adjustments using Cone-Beam Computed Tomography (CBCT) to account for patient and tumor position changes. Despite advancements in imaging and treatment delivery, patient misidentification and incorrect treatment positioning remain significant risks in radiotherapy. These errors can lead to the irradiation of non-target tissues or missing the target entirely, which can have severe consequences, including increased morbidity or failure to treat the cancer effectively.

Conventional methods of verifying patient identity and treatment accuracy often rely on manual checks, physical identifiers such as wristbands, or basic electronic systems. These methods, while useful, still leave room for human error and do not fully leverage the capabilities of modern imaging technologies. In order to be more efficient, certain clinics and medical professionals use computer models to verify patients using their medical images. However, these conventional evaluation models have also faced technical problems. For instance, conventional methods struggle with the variability and lower quality of images like CBCT compared to CT. Additionally, conventional image similarity metrics are often inadequate when directly comparing images from different modalities (CT vs. CBCT) due to differences in image resolution, noise levels, and contrast. Therefore, conventional computer-implemented solutions face technical challenges.

## SUMMARY

In accordance with a first aspect of the invention, there is provided a method as defined by claim 1.

In accordance with a second aspect of the invention, there is provided a system as defined by claim 10.

Optional features are defined by the dependent claims.

For the aforementioned reasons, there is a need for an automated, robust verification system that leverages advanced image processing techniques. There is a need for a technical solution that can evaluate different modes of medical images efficiently and accurately. What is needed is systems and methods that can reliably confirm patient identity and treatment positioning directly from imaging data, reducing the risk of human error, and enhancing overall patient safety in radiotherapy settings.

In some embodiments, the methods and systems discussed herein use an artificial intelligence model for the automated verification of patient identity and precise treatment positioning in radiotherapy. By comparing CT and CBCT (e.g., daily setup) images, the method and systems discussed herein leverage advanced image processing to ensure that treatments are accurately targeted to the correct patient and anatomical area. Designed to handle variations in image quality and robust against common discrepancies between different imaging modalities, the artificial intelligence model discussed herein can provide a reliable, efficient, and automated secondary check within existing clinical workflows. The methods and systems discussed herein significantly enhance patient safety by reducing the risk of misidentification and incorrect treatment delivery, potentially transforming practice standards in radiotherapy and other medical imaging applications.

The methods and systems discussed herein further allow for a model to be trained using unsupervised paradigms. For instance, the model discussed herein can ingest data monitored during a patient's treatment (or identification before the treatment) and use the monitored data to train itself. Therefore, the model can be operated with little to no human intervention, which is a technical advantage over conventional methods.

In some embodiments, the artificial intelligence model is a Siamese Convolutional Neural Network (SCNN) used for patient identity verification in radiotherapy. This brings multiple transformative benefits that enhance both safety and efficiency in clinical settings.

By automating the verification process using sophisticated image-matching techniques, the method and systems discussed herein minimize the risks associated with manual checks and less advanced electronic systems. This automation may ensure that the radiotherapy targets are precisely aligned with the planned treatment areas, significantly reducing the likelihood of irradiating healthy tissue.

The methods and systems discussed herein may streamline clinical workflows by providing real-time verification, which simplifies and speeds up the setup process for radiotherapy. This efficiency allows healthcare facilities to utilize their resources better and potentially treat more patients within the same time frames, thereby improving throughput and operational efficiency.

The artificial intelligence model discussed herein may be specifically designed to handle variability in image quality between different modalities, such as CT and CBCT scans in addition to 4DCT/CBCT imaging. Traditional image comparison methods often falter with such variability, but the artificial intelligence model discussed herein can maintain reliable performance, ensuring consistent verification accuracy regardless of the imaging conditions.

An additional advantage of using the method and systems discussed herein (when compared to conventional models) is the reduced dependency on physical identifiers, which are prone to human error and errors created by conventional image-comparing computer models. The artificial intelligence models discussed herein can use biometric data extracted directly from patient images, which reduces the scope for errors in patient identification that can occur with misplaced or incorrect wristbands.

An additional advantage provided by using the method and systems discussed herein (when compared to conventional models) is that the architecture of the artificial intelligence model discussed herein is adaptable and scalable, making it a versatile tool suitable for a variety of imaging setups and clinical requirements. This flexibility can ensure that the system can evolve alongside advances in imaging technology, maintaining its effectiveness without necessitating complete system replacements. That is, as the imaging modalities improve, the artificial intelligence model discussed herein can revise its training consistently, such that it applies to the latest technology. This, in contrast with conventional static technologies, allows for adaptability to new and/or improved imaging technologies.

As a secondary verification measure, the artificial intelligence models discussed herein can operate seamlessly within existing clinical protocols, adding an extra layer of verification that enhances treatment integrity without imposing additional steps on clinical staff. Therefore, the methods and systems discussed herein can be used as a retrofit measure without disrupting legacy verification systems. This allows the implementation of the methods and systems discussed herein without any downtime of the legacy system.

In some aspects, the techniques described herein relate to a method including: retrieving, by a processor, a first medical image of a patient; obtaining, by the processor using a medical imaging sensor, a second medical image of the patient; executing, by the processor, an artificial intelligence model to compare the first medical image and the second medical image of the patient, wherein the artificial intelligence model is configured to transform the first medical image and the second medical image as feature vectors into a latent space to: identify one or more features of each medical image within the latent space, and compare the one or more identified features for each medical image within the latent space to predict a distance between at least one feature of the first medical image within the latest space and at least one corresponding feature of the second medical image within the latent space, the distance associated with a likelihood that the first medical image and the second medical image belong to a same patient, transmitting, by the processor, a signal to a radiotherapy computing device indicating a warning that the first medical image and the second medical image do not belong to the same patient.

A "latent space" may refer to a multi-dimensional feature space where, for example, the fundamental characteristics of images are preserved and encoded.

In some aspects, the techniques described herein relate to a method, wherein the second medical image is obtained within a treatment room associated with the medical treatment.

In some aspects, the techniques described herein relate to a method, wherein the second medical image is obtained using the medical imaging sensor of a radiotherapy machine providing treatment to the patient.

In some aspects, the techniques described herein relate to a method, wherein the first medical image and the second medical image are both obtained within a treatment room associated with the medical treatment.

In some aspects, the techniques described herein relate to a method, wherein the first medical image and the second medical image are generated at different times.

In some aspects, the techniques described herein relate to a method, where the first medical image is a pre-treatment image of the patient.

In some aspects, the techniques described herein relate to a method, wherein the second medical image is obtained at a time after at least one treatment fraction of the patient.

In some aspects, the techniques described herein relate to a method, wherein at least one of the first medical image or the second medical image is obtained using X-ray radiography, computed tomography (CT) imaging, cone beam computed tomography (CBCT), fluoroscopy, tomosyntheses, single photon emission computed tomography (SPECT) imaging, ultrasound (US) imaging, magnetic resonance imaging (MRI), or positron emission tomography (PET) imaging.

In some aspects, the techniques described herein relate to a method, wherein the first medical image and the

**EP 4 564 365 A1**

second medical image correspond to different medical imaging modalities.

In some aspects, the techniques described herein relate to a method, wherein the first medical image and the second medical image correspond to a same medical imaging modalities.

In some aspects, the techniques described herein relate to a method, wherein the warning indicates that a wrong anatomical area of the patient is to be treated and/or that the patient is in a wrong position.

In some aspects, the techniques described herein relate to a method, wherein the first medical image has a planning target volume that is different in size than a second planning target volume depicted within the second medical image.

In some aspects, the techniques described herein relate to a method, wherein the first medical image has a planning target volume that is different in shape than a second planning target volume depicted within the second medical image.

In some aspects, the techniques described herein relate to a method, wherein the distance further indicates a visual variance between at least one anatomic feature of the first medical image compared to at least corresponding anatomic feature within the second medical image.

In some aspects, the techniques described herein relate to a method, wherein the artificial intelligence model is trained using a loss function that penalizes similar medical images with a corresponding distance that exceeds the threshold and further penalizes dissimilar medical images with a second corresponding distance that is lower than the threshold.

In some aspects, the techniques described herein relate to a system including: a non-transitory medium storing instructions that when executed cause a processor to: retrieve a first medical image of a patient; obtain using a medical imaging sensor, a second medical image of the patient; execute an artificial intelligence model to compare the first medical image and the second medical image of the patient, wherein the artificial intelligence model is configured to transform the first medical image and the second medical image as feature vectors into a latent space to: identify one or more features of each medical image within the latent space, and compare the one or more identified features for each medical image within the latent space to predict a distance between at least one feature of the first medical image within the latent space and at least one corresponding feature of the second medical image within the latent space, the distance related to a likelihood that the first medical image and the second medical image belong to a same patient, and when the distance predicted by the artificial intelligence model does not satisfy a threshold, transmit a signal to a radiotherapy computing device indicating a warning that the first medical image and the second medical image do not belong to the same patient.

Using the method and systems discussed herein, the models discussed herein can be trained to learn a projection into latent space, such that the in the latent space, the two images appear at a close distance to each other d(x1, x2) < m, and dissimilar images at a distance d(x1, x2) > m. Therefore, the model discussed herein may compare two images in a latent space and ensure that these images appear as two points that are "close" to each other (within a margin m) if they are similar, and that they appear "far" apart (outside a margin m) if the images are dissimilar (because they do not belong to the same patient).

In some aspects, the techniques described herein relate to a system, wherein the artificial intelligence model is trained using a loss function that penalizes dissimilar medical images with a corresponding distance that exceeds the threshold and further penalizes similar medical images with a second corresponding distance that is lower than the threshold.

In some aspects, the techniques described herein relate to a system, wherein at least one of the first medical image or the second medical image is obtained using X-ray radiography, computed tomography (CT) imaging, cone beam computed tomography (CBCT), fluoroscopy, tomosyntheses, single photon emission computed tomography (SPECT) imaging, ultrasound (US) imaging, magnetic resonance imaging (MRI), or positron emission tomography (PET) imaging.

In some aspects, the techniques described herein relate to a system, wherein the first medical image and the second medical image correspond to different medical imaging modalities.

In some aspects, the techniques described herein relate to a system, wherein the first medical image and the second medical image correspond to a same medical imaging modalities.

In some aspects, the techniques described herein relate to a system, wherein the second medical image is obtained within a treatment room associated with the medical treatment.

In some aspects, the techniques described herein relate to a system, wherein the second medical image is obtained using the medical imaging sensor of a radiotherapy machine providing treatment to the patient.

In some aspects, the techniques described herein relate to a system, wherein the first medical image and the second medical image are both obtained within a treatment room associated with the medical treatment.

In some aspects, the techniques described herein relate to a system, wherein the first medical image and the second medical image are generated at different times.

In some aspects, the techniques described herein relate to a system, where the first medical image is a pre-treatment image of the patient.

In some aspects, the techniques described herein relate to a system, wherein the second medical image is obtained at a time after at least one treatment fraction of the patient.

In some aspects, the techniques described herein relate to a system, wherein the warning indicates that a wrong anatomical area of the patient is to be treated and/or the patient is in a wrong position.

In some aspects, the techniques described herein relate to a system, wherein the first medical image has a planning target volume that is different in size than a second planning target volume depicted within the second medical image.

In some aspects, the techniques described herein relate to a system, wherein the first medical image has a planning target volume that is different in shape than a second planning target volume depicted within the second medical image.

In some aspects, the techniques described herein relate to a system, wherein the distance further indicates a visual variance between at least one feature of the first medical image compared to at least corresponding feature within the second medical image.

## BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.

**FIG. 1** illustrates a diagram of a system for verification of medical images, according to an embodiment.

**FIG. 2** illustrates a flow diagram of a process for verification of medical images, according to an embodiment.

**FIG. 3** illustrates an architecture of the artificial intelligence model, according to an embodiment.

**FIG. 4** illustrates an architecture of the artificial intelligence model, according to an embodiment.

**FIGS. 5A-B** illustrate a non-limiting example of the system for verification of medical images, according to an embodiment.

## DETAILED DESCRIPTION

Reference will now be made to the illustrative embodiments depicted in the drawings, and specific language will be used here to describe the same. It will nevertheless be understood that no limitation of the scope of the claims or this disclosure is thereby intended. Alterations and further modifications of the inventive features illustrated herein, and additional applications of the principles of the subject matter illustrated herein, which would occur to one skilled in the relevant art and having possession of this disclosure, are configured to be considered within the scope of the subject matter disclosed herein. Other embodiments can be used and/or other changes can be made without departing from the scope of the present disclosure. The illustrative embodiments described in the detailed description are not meant to be limiting of the subject matter presented.

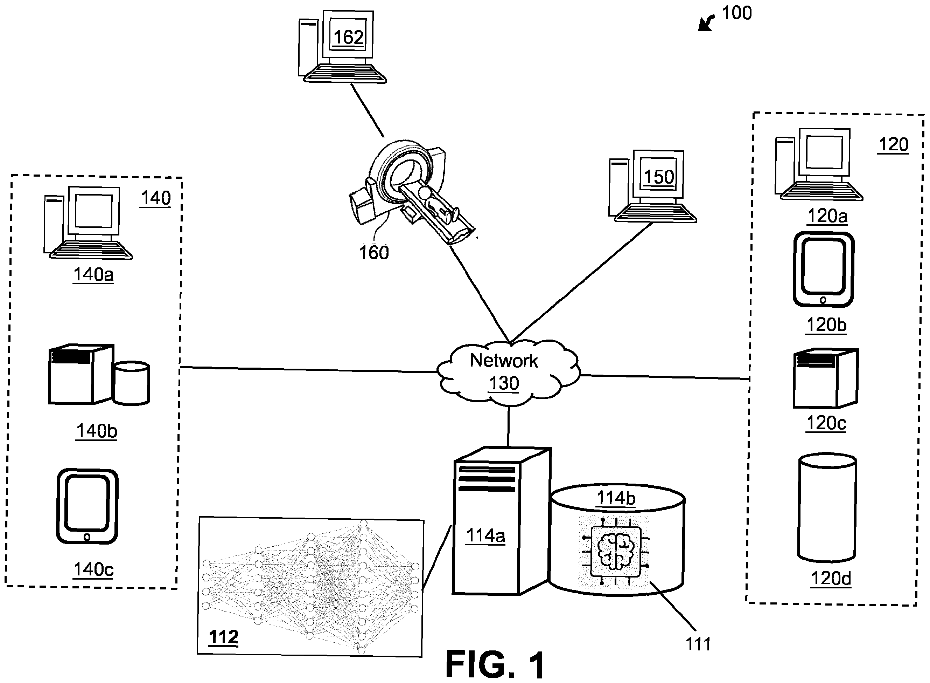

**FIG. 1** illustrates components of a system **100** for verification of medical images, according to an embodiment. The system **100** can include an analytics server **114a,** system database **114b,** a treatment planning system **111,** and electronic data sources **120a-d** (each referred to individually as an electronic data source **120** and collectively as electronic data sources **120,** unless stated otherwise), end-user devices **140a-c** (each referred to individually as an end-user device **140** and collectively as end-user devices **140,** unless stated otherwise), an administrator computing device **150,** a medical device **160,** and medical device computer(s) **162.**

Various components depicted in **FIG. 1** can belong to a radiotherapy clinic at which patients can receive radiotherapy treatment, in some cases via one or more radiotherapy machines located within the clinic (e.g., medical device **160).** The system **100** is not confined to the components described herein and can include additional or other components, not shown for brevity, which are configured to be considered within the scope of the embodiments described herein.

The above-mentioned components can be connected to each other through a network **130.** Examples of the network **130** can include, but are not limited to, private or public local-area-networks (LAN), wireless LAN (WLAN) networks, metropolitan area networks (MAN), wide-area networks (WAN), and the Internet. The network **130** can include wired and/or wireless communications according to one or more standards and/or via one or more transport mediums. The communication over the network **130** can be performed in accordance with various communication protocols such as Transmission Control Protocol and Internet Protocol (TCP/IP), User Datagram Protocol (UDP), and IEEE communication protocols. In one example, the network **130** can include wireless communications according to Bluetooth specification sets or another standard or proprietary wireless communication protocol. In another example, the network **130** can also include communications over a cellular network, including, e.g., a GSM (Global System for Mobile Communications), CDMA (Code Division Multiple Access), and EDGE (Enhanced Data for Global Evolution) network.

The analytics server **114a** can be any computing device comprising a processor and non-transitory machine-readable storage capable of executing the various tasks and processes described herein. The analytics server **114a** can employ various processors such as central processing units (CPU) and graphics processing unit (GPU), among others. Non-limiting examples of such computing devices can include workstation computers, laptop computers, server computers, and the like. While the system **100** includes a single analytics server **114a,** the analytics server **114a** can include any number of computing devices operating in a distributed computing environment, such as a cloud environment.

The analytics server **114a** can generate and display an electronic platform configured to use a treatment planning system **111** for receiving patient information, inputs from users (e.g., clinicians) such as utility functions and updated utility functions described herein, and outputting the results of execution of the treatment planning system **111.** The electronic platform can include graphical user interfaces (GUI) displayed by display devices of one or more electronic data sources **120,** the end-user devices **140,** the medical device **160,** and/or the administrator computing device **150.** An example of the electronic platform generated and hosted by the analytics server **114a** can be a web-based application or a website configured to be displayed on different electronic devices, such as mobile devices, tablets, personal computers, and the like.

The information displayed by the electronic platform can include, for example, input elements to receive data associated with a patient being treated, synchronize one or more sensors, and display results of predictions produced by the treatment planning system **111.** For instance, the analytics server **114a** can execute the treatment planning system **111** (e.g., a system such as a treatment planner that is configured and/or trained to generate fluence maps, leaf sequences, etc., as described herein for a patient being treated via the medical device **160).** The analytics server **114a** can then display the results for a clinician and/or directly revise one or more operational attributes of the medical device **160.**

The electronic data sources **120** can be any computing device comprising a processor and non-transitory machine-readable storage capable of executing the various tasks and processes described herein. For example, the electronic data sources **120** can represent various computing devices that contain, retrieve, and/or access data associated with a medical device **160,** such as data associated with operational information of currently or previously performed radiotherapy treatments (e.g., electronic log files or electronic configuration files), data associated with current and/or previously monitored patients (e.g., computed tomography (CT) scans, magnetic resonance imaging (MRI) scans, tumor locations, deformation information, and/or the like) or participants in a study, and/or the like. For instance, the analytics server **114a** can use the clinic computer **120a,** medical professional device **120b,** server **120c** (associated with a clinician and/or a clinic), and database **120d** (associated with the clinician and/or the clinic) to retrieve/receive data associated with the medical device **160.** The analytics server **114a** can retrieve the data from the end-user devices **120,** generate a dataset, and use the dataset to configure the treatment planning system **111** (e.g., models implemented by the treatment planning system **111** and/or the like). The analytics server **114a** can execute various algorithms to translate raw data received/retrieved from the electronic data sources **120** into machine-readable objects that can be stored and processed by other analytical processes as described herein.

End-user devices **140** can be any computing device comprising a processor and a non-transitory machine-readable storage medium capable of performing the various tasks and processes described herein. Non-limiting examples of an end-user device **140** can be a workstation computer, laptop computer, tablet computer, or server computer. In operation, various users such as clinicians as described herein can use end-user devices **140** to access the GUI operationally managed by the analytics server **114a** or otherwise the results of the execution of the treatment planning system 111. Specifically, the end-user devices **140** can include clinic computer **140a,** clinic server **140b,** and a medical professional device **140c.**

Even though referred to herein as "end-user" devices, these devices cannot always be operated by end-users. For instance, the clinic server **140b** cannot be directly used by an end user. However, the results stored on the clinic server **140b** can be used to populate various GUIs accessed by an end user via the medical professional device **140c.** In some embodiments, the end-user device **140** can be associated with one or more clinicians that are associated with the generation of one or more treatment plans (e.g., involved in preparing the one or more treatment plans) for patients.

The administrator computing device **150** can represent a computing device operated by a system administrator. The administrator computing device **150** can be configured to display radiotherapy treatment attributes generated by the analytics server **114a** (e.g., various analytic metrics determined during training of one or more machine learning models and/or systems); monitor various treatment planning systems **111** utilized by the analytics server **114a,** electronic data sources **120,** and/or end-user devices **140;** review feedback; and/or facilitate training or retraining (calibration) of the treatment planning system **111** that are maintained by the analytics server **114a.** In some embodiments, the methods and systems discussed herein may utilize transfer learning paradigms, such as by adapting a pre-trained machine learning model, originally trained on a broad dataset, using/leveraging the learned knowledge of the models discussed herein.

In some embodiments, the medical device **160** can be a diagnostic imaging device or a treatment delivery device.

For example, the medical device **160** can include one or more computed tomography (CT) scanners, linear accelerators (LINACs) having a multi-leaf collimator (MLC) that consists of multiple small lead leaves that can be individually moved to shape the radiation beam and deliver the dose to the tumor while minimizing the dose to surrounding healthy tissues, or other similar devices configured to transmit energy toward targeted tissue (referred to as planning target volumes) associated with a patient and, in some cases, measure the energy transferred toward the targeted tissue. The medical device **160** can also include one or more sensors configured to monitor the patient being treated. That is, the medical device **160** and/or the analytics server **114a** can be communicating with various sensors that can monitor a patient's external biological signals. Non-limiting examples of the sensors can include 3D surfacing mechanisms and optical (or other) sensors configured to monitor the patient's movements (e.g., how the patient is moving and/or breathing. In some embodiments, the medical device **160** can receive data associated with a treatment plan from the medical device computer(s) **162** that cause the medical device **160** to operate in accordance with the treatment plan.

The treatment planning system **111** can be stored in the system database **114b.** The treatment planning system **111** can be trained using data received/retrieved from the electronic data sources **120** and can be executed using data received from the end-user devices, the medical device **160,** and/or the sensor **163.** In some embodiments, the treatment planning system **111** can reside within a data repository local or specific to a clinic. In various embodiments, the treatment planning system **111** can use one or more deep learning engines to develop a treatment plan for a patient using radiation therapy. For instance, the analytics server **114a** can transmit patient attributes from the sensor **163** and execute the treatment planning system **111** accordingly. The analytics server **114a** can then display the results on one or more end-user devices **140.** In some embodiments, the analytics server **114a** can change one or more configurations of the medical device **160** based on the results predicted by the treatment planning system **111.**

The system **100** may also include an artificial intelligence model **112.** The artificial intelligence model **112** may perform the image verification and analysis. The artificial intelligence model **112** may be specifically designed and trained for the processing and analysis of medical imaging data. Initially, the artificial intelligence model **112** may engage in preprocessing the medical images, which involves refining image quality through adjustments in contrast, noise reduction, and standardization of size and resolution to ensure consistency across different imaging modalities. Following preprocessing, the artificial intelligence model **112** may utilize deep learning algorithms, such as convolutional neural networks (CNNs), to extract key features from the images. These features may represent distinctive markers capable of accurately distinguishing one patient from another. After feature extraction, these characteristics are transformed into feature vectors. This transformation encodes the nuanced anatomical features into a numerical format, compactly representing each image's essential traits. In the latent space, vectors representing similar features may cluster together while those representing dissimilar features may be positioned apart. The artificial intelligence model **112** may calculate distances between feature vectors to estimate the similarity between corresponding features across different images, thus predicting the likelihood of images belonging to the same patient.

Finally, the artificial intelligence model **112** may interpret these analytical results to provide clinical decision support, e.g., by outputting a likelihood score or categorical data, which assists healthcare professionals in confirming patient identity or aiding in diagnostic decisions.

The artificial intelligence model's **112** adaptability allows for seamless integration with various medical imaging systems and electronic health records, enabling it to be trained on diverse datasets to accommodate different medical conditions and imaging techniques such as CT scans, MRI, X-rays, fluoroscopy, tomosyntheses, and ultrasound. Even though the artificial intelligence model **112** is depicted as a single model, it could be a collection of models or sub-models. Therefore, no limitation is intended.

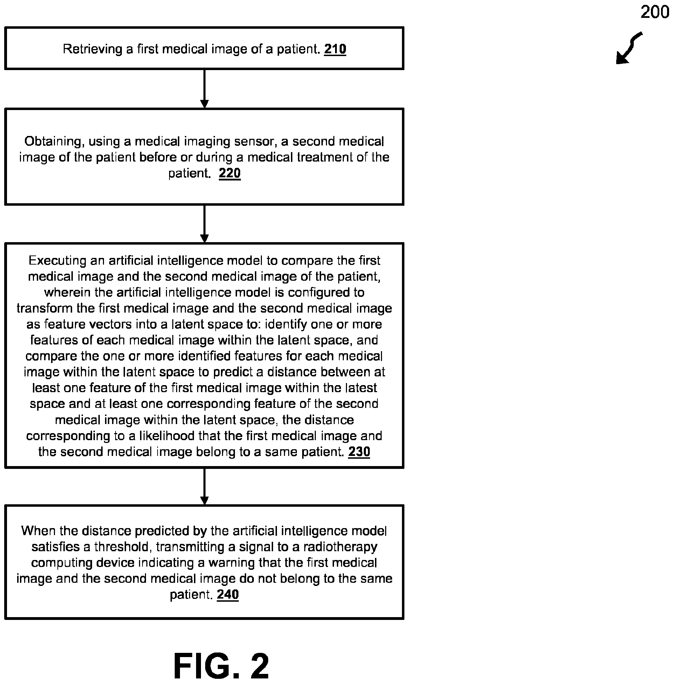

Referring to **FIG. 2,** illustrated is a flow diagram of a process **200** for verification of medical images, according to an embodiment. The process 200 may identify when a first medical image and a second medical image do not belong to the same patient, a wrong structure and/or anatomical area of the patient is to be treated, and/or the patient is in a wrong position. The process **200** includes operations **210-240.** However, other embodiments can include additional or alternative operations or can omit one or more operations altogether. The process **200** is described as being executed by an analytics server, which can be the same as, or similar to, the analytics server **114a** described in **FIG. 1.** However, one or more steps of the process **200** can be executed by any number of computing devices operating in the distributed computing system described in **FIG. 1.** For instance, one or more computing devices can locally perform part or all of the operations described in **FIG. 2.**

Radiotherapy (RT) is a proven, cost-effective method for extending the lives of cancer patients. The typical RT process begins with a Computed Tomography (CT) scan for initial treatment planning, followed by daily Cone Beam CT (CBCT) scans during treatments. CT scans, utilizing fan-shaped X-ray beams, produce detailed cross-sectional images that are crucial for pinpointing tumor locations and planning treatments. Conversely, CBCT scans generate three-dimensional images with a cone-shaped X-ray beam, offering less detail but crucial for guiding accurate radiation targeting to the tumor while protecting healthy tissue.

Despite the advancements, RT is susceptible to errors such as incorrect radiation dosing or delivery mishaps. These errors can arise at any stage, due to factors like data entry mistakes, equipment failure, or miscommunication among medical professionals. Notably, studies indicate that a significant portion of all setup errors involve incorrect patient positioning, sometimes even misidentification of the patient, leading to potentially severe or fatal consequences.

Some conventional systems use computer models for CT image comparisons to identify/verify the patient. For example, some conventional methods use gradient-based and traditional image similarity metrics to compare setup and planning CT images. However, those conventional methods have experienced a decline in accuracy when the comparison includes a CBCT image used for daily setups. This reduction is sometimes attributed to the increased noise, image artifacts, and/or reduced contrast resolution characteristic of CBCTs, which can significantly impact the effectiveness of standard similarity metrics. In some cases, hierarchical features generated by conventional convolutional neural networks (CNNs) may be resilient to these inter-modality discrepancies.

The method **200** may employ a CNN-based architecture in order to effectively bridge these quality gaps by extracting crucial anatomical landmarks essential for patient identification. The method **200** provides automated patient verification via medical imaging data. In this method, the goal is to identify the same object across different images.

At step **210,** the analytics server may retrieve a first medical image of a patient. In some embodiments, the analytics server may receive a patient identifier (e.g., name or medical record number) of the patient. Using the identifier, the analytics server may then query one or more data repositories and receive medical images of the patient. As used herein, a medical image may refer to any image that depicts one or more internal structures of the patient, such as a CT, CBCT, X-ray, MRI, ultrasound, PET scan, single photon emission computed tomography (SPECT), tomosyntheses, and/or fluoroscopy images of the patient.

In some embodiments, the image received may be present within the data repository because the medical image has been used by a medical professional for diagnosis and/or treatment planning purposes. For instance, the first medical image may refer to a CT scan of the patient that depicts the patient's tumor. An oncologist may use the CT scan for treatment planning purposes. Therefore, the first medical image may be used as a reference. In some embodiments, the reference image may be a pre-treatment medical image of the patient (e.g., before the patient receives RT treatment and/or before the patient receives a defined number of fractions of their RT treatment). As used herein, the second medical image may be two or three-dimensional.

At step **220,** the analytics server may obtain, using a medical imaging sensor, a second medical image of the patient.

The second medical image may be a medical image that has been generated before or during a medical treatment of the patient. For instance, as opposed to the first medical image, which is a pre-treatment image of the patient, the second medical image may be generated in the treatment room and before the patient's RT treatment commences. In some embodiments, the RT machine may include an imaging apparatus that is configured to generate the second medical image before executing the patient's treatment. In some embodiments, both the first and second medical images may have been produced by the same modality (e.g., RT machine) or within the same room. For instance, the first medical image may represent a CBCT of the patient after the first treatment fraction, and the second medical image may represent a CBCT of the patient before receiving the second treatment fraction.

Similar to the first medical image, the second medical image may be any medical image, such as a CT, CBCT, X-ray, SPECT, MRI, ultrasound, PET scan, radiographic images, and/or fluoroscopy image of the patient. In some embodiments, the first medical image and the second medical image may have different modalities (e.g., the first medical image is a CT, and the second medical image is a CBCT). However, in some embodiments, both medical images may belong to the same modality. As used herein, the second medical image may be two or three-dimensional.

At step **230,** the analytics server may execute an artificial intelligence model to compare the first medical image and the second medical image of the patient, wherein the artificial intelligence model is configured to transform the first medical image and the second medical image as feature vectors into a latent space to: identify one or more features of each medical image within the latent space, and compare the one or more identified features for each medical image within the latent space to predict a distance between at least one feature of the first medical image within the latent space and at least one corresponding feature of the second medical image within the latent space, the distance associated with or related to a likelihood that the first medical image and the second medical image belong to a same patient.

The analytics server may execute an artificial intelligence model that is specifically designed and trained for medical image analysis. The artificial intelligence model may perform comparative analyses between the first medical image and the second medical image of a patient in accordance with its training. The operation of the artificial intelligence model may involve the projection of the images into a latent space, designed for optimal feature analysis.

In the latent space, the artificial intelligence model may identify and isolate one or more features within each of the medical images. These features might include but are not limited to, any combination of one or more of: bone structures, organs, planning target volume, organ at risk, and any specific markers and/or and any other hidden (latent) markers relevant to patient identity verification relevant to medical diagnosis or patient identity verification. For example, the artificial intelligence model may identify features that are relevant to patient identity verification, verification of the anatomical area to be treated, and/or patient positioning verification. The artificial intelligence model may use deep learning techniques that enable the model to recognize and differentiate between such features with high precision.

Once the relevant features are identified and encoded as feature vectors in the latent space, the artificial intelligence model may compare the vectors between the two medical images. The primary objective of this comparison is to calculate the distance between corresponding features in the first and second medical images. This distance may not be merely a spatial measurement but may be an abstract representation in the model's latent space that is associated with the likelihood of both images belonging to the same patient. In some embodiments, a distance of 0 may indicate that the two images are very likely of the same patient. Moreover, a distance of greater than 0 may indicate that the two images are very unlikely of the same patient (the higher the value the more unlikely).

In order to calculate this distance, the model may use various algorithms that assess similarity or disparity based on the learned characteristics of what constitutes identity congruence within the scope of patient-specific anatomical features. The outcome of this comparison provides a probabilistic measure or likelihood, indicating whether the first and second medical images are from the same patient.

In some embodiments, the features identified by the models discussed herein and compared within the latent space may not correspond to an anatomical structure of the patient. For instance, the model may not limit its comparison to comparing latent space representation of anatomical structures. Instead, any feature from the latest space representation of one image can be compared to any corresponding feature from the second image.

Training Dataset Generation:

Before executing the artificial intelligence model, the analytics server may train the artificial intelligence model to predict a similarity distance/index between two medical images. In order to train the artificial intelligence model, the analytics server may generate a training dataset.

The training dataset utilized may encompass two and/or three-dimensional medical images (e.g., CT scans and daily setup CBCT images) of various training patients. As used herein, a training patient may refer to any person whose information may be used to train the artificial intelligence model discussed herein. Therefore, the training patient may be a previously treated patient, a patient being currently treated, and/or a participant in a medical trial.

The retrieved images may be sourced from various imaging systems, such as an internal treatment system (e.g., clinic data repositories) and/or third-party vendors. Additionally, a subset of images for a subset of the patients may be incorporated from publicly available data sources, such as the Pelvic-Reference-Data dataset. The publicly sourced images may include planning CT scans obtained using the same scanners and/or medical imaging techniques (e.g., same CBCT images captured with a particular linear accelerator). This consistency ensures that the training is performed using data that allows for more efficient prediction.

In some embodiments, to maintain ethical standards and privacy, the analytics server may execute a de-identification protocol for the collected/retrieved images. In this way, the images may be anonymized, such that various ethical and legal obligations are satisfied.

In some embodiments, for analytical clarity and experimental rigor, the images may be classified into different anatomical categories. In a non-limiting example, the medical images may be categorized into two categories of abdominal and pelvic. These categories may be further integrated into a comprehensive multi-region dataset. Each patient record in the dataset may contain at least one planning CT and one setup CBCT image, ensuring extensive coverage of the anatomical site pertinent to the study.

In some embodiments, the analytics server may be required to pair a CBCT image with a CT image matches and/or mismatches, as the data set can comprise any type of modality depending on the use-case. In some embodiments, three images may be paired instead of pairs, which may involve 1 anchor image, one positive image (same as anchor) and one negative image to create the data set. The analytics server may generate a data set that now consists of binary outcome labels (matching / mismatching) rather than just images alone. To facilitate accurate image pair matching, in some embodiments, each planning CT may be paired (e.g., labeled as such) with all corresponding CBCT images from the same patient, creating sets of matching image pairs. Conversely, non-matching image pairs may also be labeled as such where non-matching images may be generated by pairing a planning CT with a CBCT image randomly selected from a different patient. These pairings can be cataloged in order to preserve the detailed composition of each dataset subgroup. Table 1 represents a catalog of the training dataset. Specifically, Table 1 represents an overview of the body-region-specific datasets. The asterisks marks the public dataset. Therefore, this non-limiting example of the training dataset includes both private and public data.

Table 1

| Region | Patients | Matching Pairs | Mismatching Pairs | Total Dataset |
|---|---|---|---|---|
| Pelvic** | 58 | 58 | 88 | 146 |
| Pelvic | 146 | 3407 | 3527 | 6934 |

(continued)

| Region | Patients | Matching Pairs | Mismatching Pairs | Total Dataset |
|---|---|---|---|---|
| Abdominal | 75 | 4102 | 4304 | 8406 |
| Mixed | 279 | 4102 | 4304 | 8406 |

In some embodiments, the analytics server may preprocess the training data. The preprocessing phase of the medical images may involve executing various steps and protocols to standardize and enhance the data quality for better or more effective model training. Non-limiting examples of different protocols may include rigid registration, resizing, resampling, and rescaling of the image pairs. The rigid registration and resizing tasks may be performed using the proprietary 3D-3D auto-match software protocols. As a result, the CT images may be uniformly resized to defined dimensions having a defined number of pixels to align with the resolution parameters of the CBCT images or vice versa.

In some embodiments, the intensity values of the images may be rescaled to a standardized range (e.g., [-1000 to 8000] Hounsfield Units), which may be then linearly mapped to a normalized range (e.g., [0,1]). Additionally, in some embodiments, a uniformly distributed translation vector may be applied within the axial plane to all CBCT images to simulate real-world variations in patient positioning.

Training The Artificial Intelligence Model:

After the training dataset has been retrieved and pre-processed, the analytics server may use the training dataset to train the artificial intelligence model. The analytics server may use a variety of methods to train the artificial intelligence model. During training, the analytics server may facilitate the learning of feature representations from pairs of input images, enabling the model to identify and verify similarities between different imaging modalities, such as CT and CBCT scans.

The analytics server may employ a dual-branch artificial intelligence model, such as a Siamese Convolutional Neural Network (SCNN) as the core model architecture. The Siamese convolutional neural network (SCNN), which may provide a high success rate for the purposes of medical image verification are discussed herein. However, the methods and systems discussed herein are not limited to SCNN models.

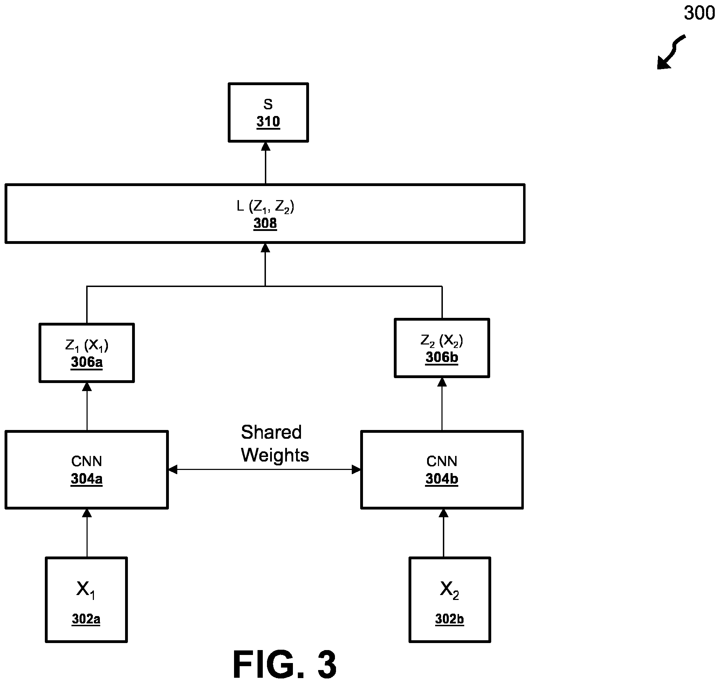

The artificial intelligence model may operate through twin CNN architectures that share weights, linked by a layer that calculates the distance (using metrics like cosine similarity or Euclidean distance) between pairs of embedded vectors, as depicted in **FIG. 3.**

As used herein, the distance may be associated with or otherwise linked to the likelihood that the first medical image and the second medical image belong to the same patient. In a non-limiting example, distances or values close to 0 may indicate a high likelihood for a matching image pair, and distances or values above 0 may indicate a decreasing likelihood of a match. For example, the greater the distance or value, the less likely there is a matching image pair (the first medical image and the second medical image belonging to the same patient).

For example, the two images x1, x2 are classified as belonging to the same patient when they are at a close distance to each other $d(x1, x2) < m$, whereas the two images are classified as belonging to different patients when they are dissimilar and are at a distance $d(x1, x2) > m$, where m is a defined threshold. In an example, images are classified as dissimilar when $d(x1, x2) \gg m$.

The depicted artificial intelligence architecture may comprise two identical sibling networks (CNN **304a** and **304b)** with shared weights and a common loss function. Specifically, the artificial intelligence model **300** may have a structure that permits the comparison of two input images (e.g., **Xi 302a** and $X_2$ **302b).** Each respective CNN may generate its own latent encoded vector **of Xi 302a** and $X_2$ **302b.** For instance, the CNN **304a** may generate Zi **306a** while the CNN **304b** may generate $Z_2$ **306b.** Each respective separate sibling network may then use its encoded vector to generate a single loss using the same loss function L $(Z_1, Z_2)$ **308.** Then using the S **310** value, a similarity between the two inputted images can be identified.

The adaptability of SCNNs to three-dimensional imaging makes them particularly effective in medical contexts, aiding in tasks such as lung nodule classification, fracture detection in CT scans, and Alzheimer's diagnosis from MRI data. Therefore, in some embodiments, the analytics server may use the SCNN architecture to authenticate patient identities in radiotherapy images, aiming to reliably ascertain that pairs of CT and CBCT scans are from the same patient. This reliable verification system may function as an automated, secondary check during the treatment setup phase, offering a cost-effective way to boost patient safety without needing extra equipment or complicating existing procedures.

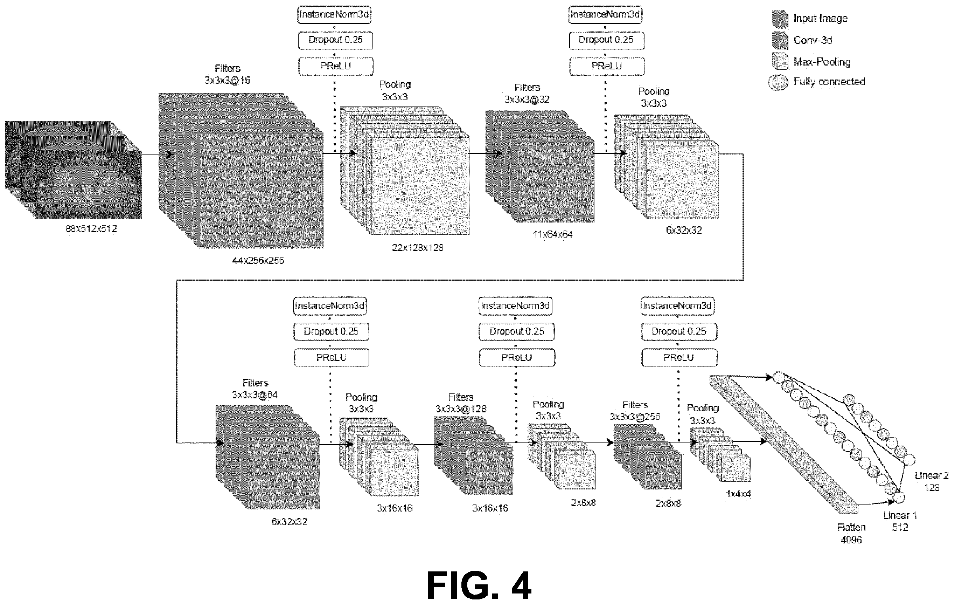

As depicted both branches are identical and operate with shared weights. In some embodiments, and as depicted in **FIG. 4,** each branch of the SCNN may be structured around a unified encoder that consists of multiple (e.g., five) layers of 3D convolution. Each convolution layer may be followed by a sequence of layers that include instance normalization, a dropout layer set to a defined dropout rate, a parametric ReLU (PReLU) activation function, and a max-pooling layer to reduce spatial dimensions while retaining important feature information. The culmination of the convolutional structure may lead to two fully connected linear layers with a defined number of nodes, respectively. This configuration may project the input images into a multi-dimensional feature space, commonly referred to as the latent space, where the fundamental characteristics of the images are preserved and encoded. **FIG. 4** provides a non-limiting example of an outline of this individual SCNN branch, illustrating the sequential process of feature extraction and reduction. In some embodiments, the CT and CBCT image within the latent space may be assessed using a contrastive loss function based on the Euclidean norm. The loss function may be used for refining the model's ability to learn discriminative embeddings that accurately represent each image's unique features, thus optimizing the model for enhanced patient identification accuracy.

In some embodiments, for uniformity and to ensure reproducibility in results, all models within this framework may be trained under consistent parameters. This may allow the SCNN to utilize robust computational resources to effectively learn and adjust to the complexities present in medical imaging data, thereby increasing the reliability and effectiveness of the patient verification process in clinical settings.

In the development of the SCNN for patient verification using medical imaging data, the model may employ a contrastive loss function. This loss function may be used to assess the similarity between pairs of input images. Using this loss function allows the model the ability to accurately verify patient identities. The contrastive loss function may be designed with a dual-purpose mechanism:

First, it may penalize scenarios where pairs of images that are supposed to be similar (e.g., from the same patient) are predicted to be too distant in the feature space.

Second, it may impose penalties on pairs of images that are dissimilar (e.g., from different patients) but appear closer than a predefined margin in the same space.

This dual penalty system may ensure that the SCNN can effectively distinguish between different patient images by pushing dissimilar images apart and pulling similar images closer in the latent space. Both image samples may be embedded into a unified latent space where this loss function is applied. The process may involve projecting the images into the space, computing the loss, and subsequently adjusting the network's weights based on the gradient of the loss, thus refining the network's ability to discriminate between patient identities. Mathematically, the loss function can be defined in the following equation:

$$L\,(x_1, x_2,\, y) = y\,.\,d\,(x_1,\, x_2)^2 + (1\text{-}y)\,.\,(\max\,(0,\, m - d\,(x_1,\, x_2))^2)$$

where L denotes the contrastive loss function. Moreover, $x_1$ and $x_2$ represent the input images, and y is a binary label that indicates whether the samples are similar (y=1) or dissimilar (y=0). The Euclidean distance between the sample embedding may be denoted by d, while m is the margin that defines the threshold below which samples are considered similar.

After training, the CT and CBCT input images may be processed and projected into a shared latent space. Within this latent space, the images may be compared using an L2 (e.g., Euclidean, Manhattan, and/or cosine distance) distance metric. This metric may incorporate a predefined cutoff threshold to effectively classify pairs of images as either "matches" (indicating they are from the same patient) or "mismatches" (indicating they are from different patients).

To assess the discriminatory properties of the model, the analytics server may use a Receiver Operator Characteristic (ROC) analysis. As used herein, the ROC may refer to a protocol used to evaluate the diagnostic ability of binary classifiers. Using ROC, the analytics server may plot the true positive rate (sensitivity) against the false positive rate (1-specificity) at various threshold settings, providing a comprehensive overview of the model's performance across different decision thresholds.

In some embodiments, a Youden index may be used to evaluate the model's performance. Using this index may allow the analytics server to determine the optimal cutoff value, often referred to as the Jaccard cutoff, where the sum of sensitivity and specificity is maximized, thus providing the best balance between true positive and true negative identification rates. The selected threshold derived from the Youden index may be further validated through the calculation of the macro average Fi score on an independent holdout-validation set. The $F_1$ score may refer to a harmonic mean of precision and recall and serves as a critical metric for evaluating the accuracy of the classifier, particularly when the data classes are imbalanced. These metrics provide additional insights into the model's ability to correctly identify matches and mismatches, respectively, which can be used to properly calibrate the model. This detailed analytical approach ensures that the model is not only effective but also reliable and adaptable to various clinical settings, ultimately enhancing patient safety through improved verification processes.

Despite the distinct differences in quality between CT and CBCT imaging modalities, the artificial intelligence model discussed herein can effectively verify patient identity using unique anatomical features. The model provides a robust translation invariance across the displacement ranges tested during training, making it particularly suitable for clinical settings where minimal positioning errors can occur during treatment setup.

Referring back to **FIG. 2,** at step **240,** the analytics server may, when the distance predicted by the artificial intelligence model satisfies a threshold, transmit a signal to a radiotherapy computing device indicating a warning that the first medical image and the second medical image do not belong to the same patient.

After executing the model, the analytics server may receive an indication that the first medical image is sufficiently similar to the second medical image. Specifically, the distance representing a similarity between the two images may be evaluated. If the distance satisfies a threshold (e.g., the distance is less than a defined threshold), the analytics server may determine that the images belong to the same patient. If the distance does not satisfy a threshold, the analytics server may transmit a warning notification to a radiotherapy machine associated with treating the patient, for example indicating that the images probably belong to different patients.

The artificial intelligence model discussed herein may be able to determine that the two medical images belong to the same patient, even if the PTV/tumor is different in size or shape.

In the evaluation process conducted by the artificial intelligence model, differences in the shape, size, or other visual attributes of a tumor in the two medical images being analyzed may be observed. These variations are often indicative of the treatment's impact on the tumor. As treatments like chemotherapy or radiation therapy progress, they can cause significant changes in the tumor's characteristics, which are captured in sequential medical images. Moreover, in some cases, patients lose significant weight after receiving one or more fractions of treatments. The artificial intelligence model may be specially trained to detect and analyze these changes, attributing differences not to discrepancies in patient identity but to the natural course of treatment response. For example, the artificial intelligence model may be trained to identify when differences in the shape, size, or other visual attributes of aspects of two medical images are due to (i) a difference in patient identity or (ii) a natural course of treatment response.

Though certain aspects of certain embodiments are discussed in relation to CT and CBCT images, it is expressly noted that the methods and systems discussed herein apply to all medical images. For instance, the artificial intelligence model discussed herein could be adapted for use in MRI-only workflows. The model may also be used for refining treatment positioning when CBCT scans replace planning CT scans. In some embodiments, by identifying the closest anatomical matches, the model could help streamline the creation of dosage plans and treatment setups for new patients with similar body types and structures. Such applications could significantly enhance operational efficiency, potentially reducing time spent on manual adjustments and increasing the overall treatment capacity of a healthcare facility. These promising avenues highlight the versatility of the SCNN and its potential to transform various aspects of clinical practice.

In some embodiments, the methods and systems discussed herein can be used to find similar patients (e.g., patients with similar anatomy) for various data science-related tasks and model building. For instance, when curating a training dataset, the method and systems discussed herein can be used to identify similar patients. In another example, a previously treated patient with sufficient similarity to a patient being treated may be identified, such that the treatment planning can be compared with the previously treated patient.

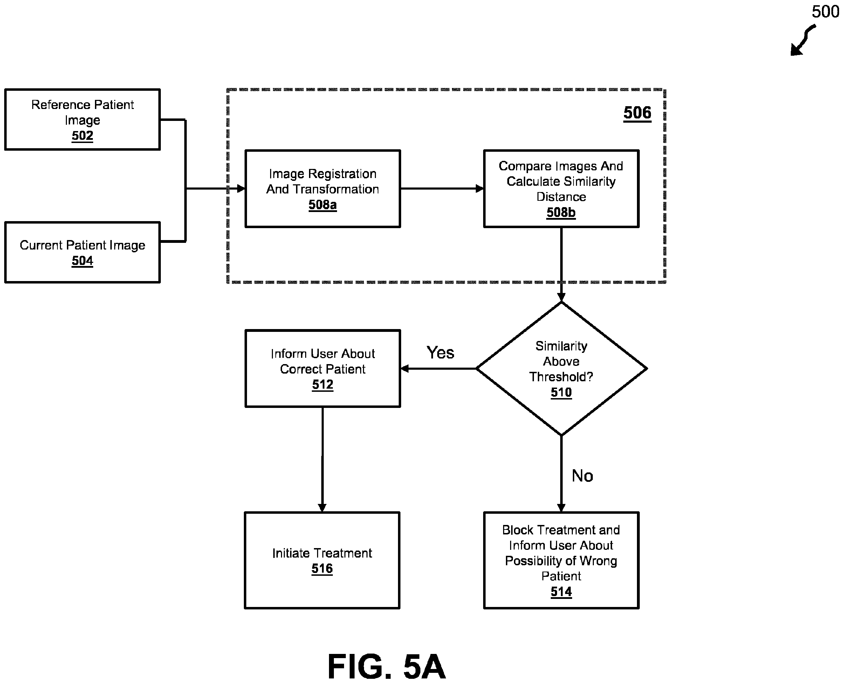

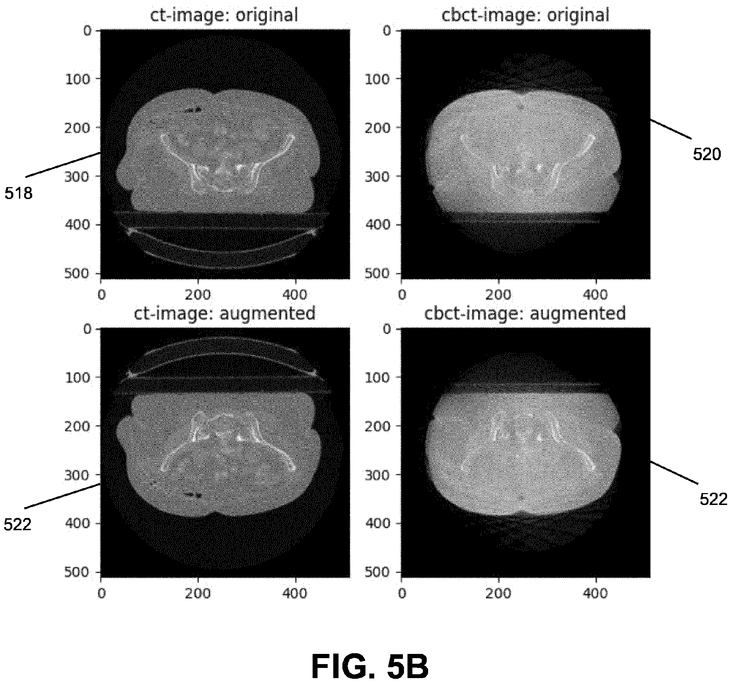

Referring now to **FIGS. 5A-B**, a non-limiting example of the operation of the methods and systems discussed herein. An example **500** illustrates how the artificial intelligence model discussed herein can be used. In the example **500,** the analytics server receives an indication that a patient is about to receive RT treatment. As a result, the analytics server retrieves a reference patient image from an internal database using the patient's name (step **502**). The reference image may be a CT scan of the patient that illustrates the patient's PTV, such as the medical image **518** in **FIG. 5B**. The analytics server may then obtain a current medical image of the patient (step **504**). For instance, the current patient image may be a CBCT of the patient, generated within the treatment room, e.g., using an imaging apparatus of the RT machine. For instance, the analytics server may obtain the medical image **520** in **FIG. 5B.**

The analytics server may then execute the artificial intelligence model discussed herein (step **506**). The artificial intelligence model may perform image registration and transformation (step **508a).** In some embodiments, during model training, certain images may be augmented as depicted in **FIG. 5B (518-522).** However, the finalized model may not involve image augmentation during comparison. The transformation may rather include the rescaling and normalization of the images during preprocessing and projecting the image into the latent space.

The analytics server may then evaluate the similarity distance in the step **510**. If the similarity satisfies the threshold (e.g., yes branch in **FIG. 5A),** then the analytics server may transmit a notification to a computer device of an administrator and inform the medical professional that the patient about to be treated is the correct patient (step **512**). The analytics server may also initiate the treatment by instructing the RT machine to commence treatment of the patient (step **516).**

As used herein, satisfying a threshold may refer to a value exceeding (e.g., is above) the threshold. In some embodiments, satisfying a threshold may refer to a value that is below the threshold. For instance, a distance associated with or otherwise related to a similarity of two structures may satisfy a threshold, when its value is below the threshold.

If the similarity does not satisfy the threshold (e.g., the "No" branch from step 510 in **FIG. 5A),** then the analytics server may block the treatment (step **514**). Specifically, the analytics server may transmit an instruction to the RT machine and stop any impending treatments. The analytics server may also transmit a warning notification to one or more computers operated by the medical professionals and indicate that the patient about to be treated is the wrong patient, wrong anatomical area of the right patients, and/or wrong position of the right patient.

In some embodiments, the patient may be the right patient. However, the treatment area may be determined to be incorrect. For instance, if the PTV is identified in the original CT scan of the patient but not included within the CBCT, the warning message may indicate that the wrong structure (e.g., anatomical area) of the patient or a wrong position of the patient is being radiated.

The various illustrative logical blocks, modules, circuits, and algorithm steps described in connection with the embodiments disclosed herein can be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans can implement the described functionality in varying ways for each particular application, but such implementation decisions should not be interpreted as causing a departure from the scope of this disclosure or the claims.

Embodiments implemented in computer software can be implemented in software, firmware, middleware, microcode, hardware description languages, or any combination thereof. A code segment or machine-executable instructions can represent a procedure, a function, a subprogram, a program, a routine, a subroutine, a module, a software package, a class, or any combination of instructions, data structures, or program statements. A code segment can be coupled to another code segment or a hardware circuit by passing and/or receiving information, data, arguments, parameters, or memory contents. Information, arguments, parameters, data, etc., can be passed, forwarded, or transmitted via any suitable means, including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions can be stored as one or more instructions or code on a non-transitory computer-readable or processor-readable storage medium. The steps of a method or algorithm disclosed herein can be embodied in a processor-executable software module, which can reside on a computer-readable or processor-readable storage medium. A non-transitory computer-readable or processor-readable media includes both computer storage media and tangible storage media that facilitate the transfer of a computer program from one place to another. A non-transitory processor-readable storage media can be any available media that can be accessed by a computer. By way of example, and not limitation, such non-transitory processor-readable media can comprise RAM, ROM, EEPROM, CD-ROM or other optical disk storage, magnetic disk storage or other magnetic storage devices, or any other tangible storage medium that can be used to store desired program code in the form of instructions or data structures and that can be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm can reside as one or any combination or set of codes and/or instructions on a non-transitory processor-readable medium and/or computer-readable medium, which can be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein can be applied to other embodiments without departing from the scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope being indicated by the following claims.

CLAUSES

The following numbered clauses are subject-matter of, but not currently claims of, the present EP application. **That is, the following clauses are part of the description, and are not claims.** The Applicant reserves the right to claim this subject-matter in this EP application.

    1. A method comprising:

retrieving, by a processor, a first medical image of a patient;

obtaining, by the processor using a medical imaging sensor, a second medical image of the patient;

executing, by the processor, an artificial intelligence model to compare the first medical image and the second medical image of the patient, wherein the artificial intelligence model is configured to transform the first medical image and the second medical image as feature vectors into a latent space to:

identify one or more features of each medical image within the latent space, and compare the one or more identified features for each medical image within the latent space to predict a distance between at least one feature of the first medical image within the latent space and at least one corresponding feature of the second medical image within the latent space, the distance associated with a likelihood that the first medical image and the second medical image belong to a same patient; and

when the distance predicted by the artificial intelligence model does not satisfies a threshold, transmitting, by the processor, a signal to a radiotherapy computing device indicating a warning that the first medical image and the second medical image do not belong to the same patient.

2. The method of clause 1, wherein the second medical image is obtained within a treatment room associated with the medical treatment.

3. The method of clause 1, wherein the second medical image is obtained using the medical imaging sensor of a radiotherapy machine providing treatment to the patient.

4. The method of clause 1, wherein the first medical image and the second medical image are both obtained within a treatment room associated with the medical treatment.

5. The method of clause 1, wherein the first medical image and the second medical image are generated at different times.

6. The method of clause 1, where the first medical image is a pre-treatment image of the patient.

7. The method of clause 1, wherein the second medical image is obtained at a time after at least one treatment fraction of the patient.

8. The method of clause 1, wherein at least one of the first medical image or the second medical image is obtained using X-ray radiography, computed tomography (CT) imaging, cone beam computed tomography (CBCT), fluoroscopy, tomosyntheses, single photon emission computed tomography (SPECT) imaging, ultrasound (US) imaging, magnetic resonance imaging (MRI), or positron emission tomography (PET) imaging.

9. The method of clause 1, wherein the first medical image and the second medical image correspond to different medical imaging modalities.

10. The method of clause 1, wherein the first medical image and the second medical image correspond to a same medical imaging modalities.

11. The method of clause 1, wherein the warning indicates that a wrong anatomical area of the patient is to be treated the patient is in a wrong position.

12. The method of clause 1, wherein the first medical image has a planning target volume that is different in size than a second planning target volume depicted within the second medical image.

13. The method of clause 1, wherein the first medical image has a planning target volume that is different in shape than a second planning target volume depicted within the second medical image.

14. The method of clause 1, wherein the distance further indicates a visual variance between at least one feature of the first medical image compared to at least corresponding feature within the second medical image.

15. The method of clause 1, wherein the artificial intelligence model is trained using a loss function that penalizes similar medical images with a corresponding distance that exceeds the threshold and further penalizes dissimilar medical images with a second corresponding distance that is lower than the threshold.

16. A system comprising:
a non-transitory medium storing instructions that when executed cause a processor to:

retrieve a first medical image of a patient;
obtain using a medical imaging sensor, a second medical image of the patient;
execute an artificial intelligence model to compare the first medical image and the second medical image of the patient, wherein the artificial intelligence model is configured to transform the first medical image and the second medical image as feature vectors into a latent space to:

identify one or more features of each medical image within the latent space, and compare the one or more identified features for each medical image within the latent space to predict a distance between at least one feature of the first medical image within the latent space and at least one corresponding feature of the second medical image within the latent space, the distance associated with a likelihood that the first medical image and the second medical image belong to a same patient;
when the distance predicted by the artificial intelligence model does not satisfy a threshold, transmit a signal to a radiotherapy computing device indicating a warning that the first medical image and the second medical image do not belong to the same patient.

17. The system of clause 16, wherein the artificial intelligence model is trained using a loss function that penalizes similar medical images with a corresponding distance that satisfies the threshold and further penalizes dissimilar medical images with a second corresponding distance that is lower than the threshold.

18. The system of clause 16, wherein at least one of the first medical image or the second medical image is obtained using X-ray radiography, computed tomography (CT) imaging, cone beam computed tomography (CBCT), fluoroscopy, tomosyntheses, single photon emission computed tomography (SPECT) imaging, ultrasound (US) imaging, magnetic resonance imaging (MRI), or positron emission tomography (PET) imaging.

19. The system of clause 16, wherein the first medical image and the second medical image correspond to different medical imaging modalities.

20. The system of clause 16, wherein the first medical image and the second medical image correspond to a same medical imaging modalities.

**Claims**

1. A method comprising:

retrieving, by a processor, a first medical image of a patient;
obtaining, by the processor using a medical imaging sensor, a second medical image of the patient;
executing, by the processor, an artificial intelligence model to compare the first medical image and the second medical image of the patient, wherein the artificial intelligence model is configured to transform the first medical image and the second medical image as feature vectors into a latent space to:

identify one or more features of each medical image within the latent space, and
compare the one or more identified features for each medical image within the latent space to predict a distance between at least one feature of the first medical image within the latent space and at least one corresponding feature of the second medical image within the latent space, the distance associated with a level of correspondence between the first medical image and the second medical image; and

when the distance predicted by the artificial intelligence model does not satisfy a threshold, transmitting, by the processor, a signal to a radiotherapy computing device indicating a warning.

2. The method of claim 1 wherein the distance is associated with a likelihood that the first medical image and the second medical image belong to a same patient;
and optionally:
when the distance predicted by the artificial intelligence model does not satisfy a threshold, transmitting, by the processor, a signal to a radiotherapy computing device indicating a warning that the first medical image and the second medical image do not belong to the same patient.

3. The method of claim 1 wherein the distance is associated with a likelihood that the first medical image and the second medical image relate to different structures, anatomical areas, and/or patient positions;
   and optionally:
   when the distance predicted by the artificial intelligence model does not satisfy a threshold, transmitting, by the processor, a signal to a radiotherapy computing device indicating a warning that (i) a wrong structure and/or anatomical area of the patient is to be treated, and/or (ii) the patient is in a wrong position.

4. The method of any preceding claim, wherein the second medical image is obtained within a treatment room associated with the medical treatment;
   and/or:
   wherein the second medical image is obtained using the medical imaging sensor of a radiotherapy machine providing treatment to the patient.

5. The method of any preceding claim, wherein the first medical image and the second medical image are both obtained within a treatment room associated with the medical treatment;
   and/or:
   wherein the first medical image and the second medical image are generated at different times.

6. The method of any preceding claim, where the first medical image is a pre-treatment image of the patient;
   and/or:
   wherein the second medical image is obtained at a time after at least one treatment fraction of the patient.

7. The method of any preceding claim, wherein at least one of the first medical image or the second medical image is obtained using X-ray radiography, computed tomography (CT) imaging, cone beam computed tomography (CBCT), fluoroscopy, tomosyntheses, single photon emission computed tomography (SPECT) imaging, ultrasound (US) imaging, magnetic resonance imaging (MRI), or positron emission tomography (PET) imaging;
   and optionally one of:

   (i) wherein the first medical image and the second medical image correspond to different medical imaging modalities; and:
   (ii) wherein the first medical image and the second medical image correspond to a same medical imaging modality.

8. The method of any preceding claim, wherein the first medical image has a planning target volume that is different in size than a second planning target volume depicted within the second medical image;
   and/or:
   wherein the first medical image has a planning target volume that is different in shape than a second planning target volume depicted within the second medical image.

9. The method of any preceding claim, wherein the distance further indicates a visual variance between at least one feature of the first medical image compared to at least corresponding feature within the second medical image;
   and/or:
   wherein the artificial intelligence model is trained using a loss function that penalizes similar medical images with a corresponding distance that exceeds the threshold and further penalizes dissimilar medical images with a second corresponding distance that is lower than the threshold.

10. A system comprising:

   a non-transitory medium storing instructions that when executed cause a processor to:

      retrieve a first medical image of a patient;
      obtain using a medical imaging sensor, a second medical image of the patient;
      execute an artificial intelligence model to compare the first medical image and the second medical image of the patient, wherein the artificial intelligence model is configured to transform the first medical image and the second medical image as feature vectors into a latent space to:

identify one or more features of each medical image within the latent space, and compare the one or more identified features for each medical image within the latent space to predict a distance between at least one feature of the first medical image within the latent space and at least one corresponding feature of the second medical image within the latent space, the distance associated with a level of correspondence between the first medical image and the second medical image;
and
when the distance predicted by the artificial intelligence model does not satisfy a threshold, transmit a signal to a radiotherapy computing device indicating a warning.

11. The system of claim 10, wherein the distance is associated with a likelihood that the first medical image and the second medical image belong to a same patient;
and optionally wherein the system is further configured to:
when the distance predicted by the artificial intelligence model does not satisfy a threshold, transmit a signal to a radiotherapy computing device indicating a warning that the first medical image and the second medical image do not belong to the same patient.

12. The system of claim 10 wherein the distance is associated with a likelihood that the first medical image and the second medical image relate to different structures, anatomical areas, and/or patient positions;
and optionally wherein the system is further configured to:
when the distance predicted by the artificial intelligence model does not satisfy a threshold, transmit a signal to a radiotherapy computing device indicating a warning that (i) a wrong structure and/or anatomical area of the patient is to be treated, and/or (ii) the patient is in a wrong position.

13. The system of any of claim 10 to claim 12, wherein the artificial intelligence model is trained using a loss function that penalizes similar medical images with a corresponding distance that satisfies the threshold and further penalizes dissimilar medical images with a second corresponding distance that is lower than the threshold.

14. The system of any of claim 10 to claim 13, wherein at least one of the first medical image or the second medical image is obtained using X-ray radiography, computed tomography (CT) imaging, cone beam computed tomography (CBCT), fluoroscopy, tomosyntheses, single photon emission computed tomography (SPECT) imaging, ultrasound (US) imaging, magnetic resonance imaging (MRI), or positron emission tomography (PET) imaging.

15. The system of any of claim 10 to claim 14, wherein the first medical image and the second medical image correspond to different medical imaging modalities;
and/or:
wherein the first medical image and the second medical image correspond to a same medical imaging modalities.

**FIG. 1**

EP 4 564 365 A1

200

```
┌─────────────────────────────────────────────────────┐
│   Retrieving a first medical image of a patient. 210 │
└─────────────────────────────────────────────────────┘
                          │
                          ▼
┌─────────────────────────────────────────────────────┐
│   Obtaining, using a medical imaging sensor, a second │
│   medical image of the patient before or during a     │
│   medical treatment of the patient. 220               │
└─────────────────────────────────────────────────────┘
                          │
                          ▼
┌─────────────────────────────────────────────────────┐
│   Executing an artificial intelligence model to       │
│   compare the first medical image and the second      │
│   medical image of the patient, wherein the           │
│   artificial intelligence model is configured to      │
│   transform the first medical image and the second    │
│   medical image as feature vectors into a latent       │
│   space to: identify one or more features of each      │
│   medical image within the latent space, and           │
│   compare the one or more identified features for      │
│   each medical image within the latent space to        │
│   predict a distance between at least one feature of    │
│   the first medical image within the latest space and   │
│   at least one corresponding feature of the second     │
│   medical image within the latent space, the distance   │
│   corresponding to a likelihood that the first medical  │
│   image and the second medical image belong to a        │
│   same patient. 230                                     │
└─────────────────────────────────────────────────────┘
                          │
                          ▼
┌─────────────────────────────────────────────────────┐
│   When the distance predicted by the artificial        │
│   intelligence model satisfies a threshold,            │
│   transmitting a signal to a radiotherapy computing    │
│   device indicating a warning that the first medical   │
│   image and the second medical image do not belong     │
│   to the same patient. 240                             │
└─────────────────────────────────────────────────────┘
```

# FIG. 2

**FIG. 3**

**FIG. 4**

**FIG. 5A**

**FIG. 5B**

EP 4 564 365 A1

Europäisches
Patentamt

European
Patent Office

Office européen
des brevets

# EUROPEAN SEARCH REPORT

**Application Number**

EP 24 21 6166

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/251099 A1 (KUNZ PATRIK [CH] ET AL) 26 September 2013 (2013-09-26) | 1-8,11, 12,14,15 | INV.<br>G16H30/40 |
| Y | * paragraph [0012] - paragraph [0014] *<br>* paragraph [0020] - paragraph [0021] *<br>* paragraph [0025] - paragraph [0026] *<br>* paragraph [0032] *<br>* figure 2 * | 9,13 | G16H20/40<br>G06N3/045<br>G06T7/00 |
| Y | KIM BACH NGOC ET AL: "Privacy-Net: An Adversarial Approach for Identity-Obfuscated Segmentation of Medical Images",<br>IEEE TRANSACTIONS ON MEDICAL IMAGING, IEEE, USA,<br>vol. 40, no. 7, 12 March 2021 (2021-03-12) , pages 1737-1749, XP011863627,<br>ISSN: 0278-0062, DOI: 10.1109/TMI.2021.3065727<br>[retrieved on 2021-06-30]<br>* abstract *<br>* figure 1(a) *<br>* page 1739 - page 1742 *<br>* Section III. METHODOLOGY * | 9,13 | |
| A | US 2004/101180 A1 (DOI KUNIO [US] ET AL) 27 May 2004 (2004-05-27)<br>* figure 1 *<br>* paragraph [0035] - paragraph [0038] * | 1-15 | |
| A | WITOLD OLESZKIEWICZ ET AL: "Siamese Generative Adversarial Privatizer for Biometric Data",<br>ARXIV.ORG, CORNELL UNIVERSITY LIBRARY, 201 OLIN LIBRARY CORNELL UNIVERSITY ITHACA, NY 14853,<br>23 April 2018 (2018-04-23), XP081421267,<br>* the whole document * | 1-15 | |

**TECHNICAL FIELDS SEARCHED (IPC)**

G16H
G06T
G06N

The present search report has been drawn up for all claims

| Place of search | Date of completion of the search | Examiner |
|---|---|---|
| Munich | 18 March 2025 | Hauber, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 6166

This annex lists the patent family members relating to the patent documents cited in the above-mentioned European search report.
The members are as contained in the European Patent Office EDP file on
The European Patent Office is in no way liable for these particulars which are merely given for the purpose of information.

18-03-2025

| Patent document cited in search report | Publication date | Patent family member(s) | Publication date |
|---|---|---|---|
| US 2013251099 A1 | 26-09-2013 | CN 103315746 A<br>US 2013251099 A1 | 25-09-2013<br>26-09-2013 |
| US 2004101180 A1 | 27-05-2004 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63604882